(19) Europäisches Patentamt
European Patent Office
Office européen des brevets

(11) Veröffentlichungsnummer : **0 330 999 B1**

(12) **EUROPÄISCHE PATENTSCHRIFT**

(45) Veröffentlichungstag der Patentschrift : **09.09.92 Patentblatt 92/37**

(51) Int. Cl.[5] : **C07C 33/02,** C07C 29/36

(21) Anmeldenummer : **89103147.8**

(22) Anmeldetag : **23.02.89**

(54) **Verfahren zur Herstellung von Octadienolen.**

(30) Priorität : **27.02.88 DE 3806305**

(43) Veröffentlichungstag der Anmeldung : **06.09.89 Patentblatt 89/36**

(45) Bekanntmachung des Hinweises auf die Patenterteilung : **09.09.92 Patentblatt 92/37**

(84) Benannte Vertragsstaaten : **BE DE ES FR GB IT NL**

(56) Entgegenhaltungen :
**EP-A- 0 287 066**
**DE-B- 2 011 163**
**DE-B- 2 018 054**
**GB-A- 2 074 156**

(56) Entgegenhaltungen :
**CHEMICAL ABSTRACTS, Band 80, Nr. 11, 18. März 1974, Columbus, Ohio, USA; T. MITSUYASU et al: "2,7-Octadienol from butadiene",Seite 319, Zusammenfassung Nr. 59 446e**
**SOVIET INVENTIONS ILLUSTRATED, Sektion Ch, Woche 8340, 16. 11. 1983, Derwent Publications Ltd., London, D 23**
**PATENT ABSTRACTS OF JAPAN, unexamined applications, C Sektion, Band 3, Nr. 23, 26. 2. 1979; THE PATENT OFFICE JAPANESEGOVERMENT, Seite 55 C 38**

(73) Patentinhaber : **BASF Aktiengesellschaft**
**Carl-Bosch-Strasse 38**
**W-6700 Ludwigshafen (DE)**

(72) Erfinder : **Roeper, Michael, Dr.**
**Albert-Schweitzer-Strasse 10**
**W-6706 Wachenheim (DE)**
Erfinder : **Bertleff, Werner, Dr.**
**Franz-Marc-Strasse 12**
**W-6806 Viernheim (DE)**
Erfinder : **Koeffer, Dieter, Dr.**
**Neuhoefer Strasse 30**
**W-6700 Ludwigshafen (DE)**

EP 0 330 999 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Octadienolen durch Telomerisation von Buta-1,3-dien (Butadien) mit Wasser in Gegenwart von Kohlendioxid und eines Katalysatorsystems aus einer Palladiumverbindung und einer tertiären Phosphorverbindung.

Octadienole sind unter anderem als Zwischenprodukte zur Herstellung von Octylalkoholen verwendbar, die ihrerseits zur Herstellung von Weichmachern wie Dioctylphthalat dienen. Da hierbei das Octan-1-ol bevorzugt wird, haben solche Octadienole die größte Bedeutung, die sich wie Octa-2,7-dien-1-ol in Octan-1-ol überführen lassen, jedoch sind gerade diese Octadienole bisher nur in unzureichenden Ausbeuten erhältlich.

Aus der DE-A-20 18 054 ist es bekannt, daß die Telomerisation von Butadien mit Wasser in Gegenwart von Kohlendioxid, eines Lösungsmittels sowie eines Katalysatorsystems aus einer Palladium(O)- oder (II)-Verbindung und eines tertiären Phosphins oder Phosphits Octadienole ergibt

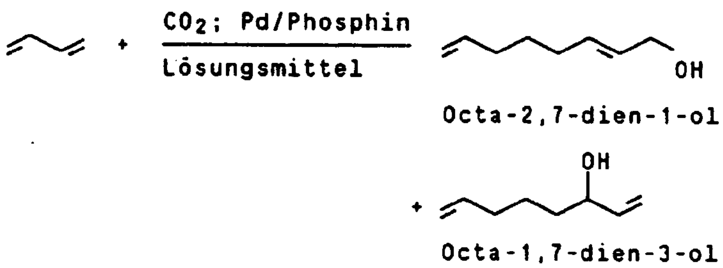

Octa-2,7-dien-1-ol
Octa-1,7-dien-3-ol

wobei man Octadienylether und Polyene wie Octa-1,3,7-trien als Nebenprodukte erhält.

Die hierbei erzielbaren Ausbeuten an den Octadienolen sowie auch die Selektivität bezüglich des Octa-2,7-dien-1-ols sind jedoch unbefriedigend.

Des weiteren liefert die Telomerisation von Butadien mit Wasser in Gegenwart von Palladium-(II)-diacetat und Triphenylphosphin als Katalysatorsystem sowie von Ameisensäure und Dioxan Octadienole (CA 80, 59446e (1974), JP-B 73/78107). Nachteile dieser Umsetzung sind die geringe Gesamtausbeute von 62 % an Octadienolen sowie die lange Reaktionsdauer von 17 Stunden.

Der Erfindung lag daher die Aufgabe zugrunde, die geschilderten Nachteile zu beheben und vor allem das Octa-2,7-dien-1-ol in höheren Ausbeuten als bisher zugänglich zu machen.

Demgemäß wurde ein Verfahren zur Herstellung von Octadienolen durch Telomerisation von Buta-1,3-dien mit Wasser in Gegenwart von Kohlendioxid und eines Katalsatorsystems aus einer Palladiumverbindung und einer tertiären Phosphorverbindung gefunden, welches dadurch gekennzeichnet ist, daß man es in Gegenwart einer Säure ausgewählt aus der Gruppe enthaltend Tetrafluorborsäure, Hexafluorphosphorsäure, Methansulfonsäure, Trifluormethansulfonsäure, p-Toluolsufonsäure, Schwefelsäure, Trifluoressigsäure, Trichloressigsäure und Essigsäure durchführt, wobei die Konzentration der Säure 0,1 bis 150 Äquivalent-% pro Mol Palladium beträgt.

Die für das erfindungsgemäße Verfahren in Betracht kommenden Säuren sind als nicht-koordinierende Säuren zu verstehen, deren Anionen mit Palladium keine stabilen Komplexverbindung eingehen. Näheres zur Theorie der nicht-koordinierenden Säuren ist dem Lehrbuch von F.A. Cotton und G. Wilkinson, Anorganische Chemie, 3. Auflage, Verlag Chemie, Interscience Publishers, Seiten 179, 238, 394 und 506 zu entnehmen.

Ungeeignete, da koordinierende Säuren, sind in erster Linie Halogenwasserstoffsäuren wie HCl, weil deren Anionen mit Palladium stabile Komplexe des Typs $[PdCl_4]^{2-}$, $[Pd_2Cl_6]^{2-}$ oder $[PdCl_3L]^{-}$ bilden, wobei L ein Ligand wie ein tertiäres Phosphin oder Phosphit ist, denn derartige stabile Komplexe haben keine katalytische Aktivität mehr, wie sie für die Butadientelomerisation erforderlich ist. Welche Säuren im Einzelfall geeignet sind und welche nicht, läßt sich anhand der gegebenen Lehre unschwer, gegebenenfalls mittels einiger Vorversuche ermitteln.

Wenngleich die Wirkungsweise der Säure noch nicht bekannt ist, so ist doch anzunehmen, daß das Proton der Säure mit dem $\pi$-Allylsystem des Butadien-Palladium-Komplexes ein Kation bildet, an welches sich leichter eine Hydroxylgruppe anlagert als an das elektrisch neutrale System.

Das Verhältnis von Säure zu Palladium beträgt im allgemeinen 0,1 bis 150, vorzugsweise 50 bis 100 Äquivalent-% pro Mol Palladium.

Zur Bereitung des Katalysatorsystems eignen sich grundsätzlich alle löslichen Palladium-(O)- und Palladium-(II)-Verbindungen mit Ausnahme stabiler Komplexverbindungen vom oben erwähnten Typ und solcher Verbindungen, beispielsweise von Palladiumhalogeniden, welche in derartige stabile Komplexe übergehen können. Als Palladiumverbindungen kommen beispielsweise

$Pd(OAc)_2$,
$Pd(PPh_3)_4$,
$Pd(dba)_2$ (dba = Dibenzylidenaceton),
$[Pd(acac)(PPh_3)_2]BF_4$(acac = Acetylaceton)
$[Pd(h^3\text{-}C_3H_5)(COD)]BF_4$(COD = 1,5-Cyclooctadien) und
$[Pd(acac)(COD)]BF_4$
sowie in erster Linie Palladium-(II)-acetylacetonat in Betracht.

Die Menge der Pd-Verbindungen ist nicht kritisch, beträgt jedoch vorzugsweise $10^{-5}$ bis 0,1, besonders $10^{-4}$ bis $10^{-2}$ mol Palladium pro Mol Butadien. Die tertiären Phosphorverbindungen fungieren als stabilisierende Liganden L in den aktiven Palladiumkomplexen des Typs

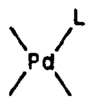 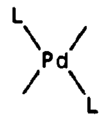 und 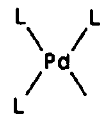

Als Liganden L eignen sich prinzipiell alle Phosphine und Phosphite, z.B. Trialkyl- und Triarylphosphine und -phosphite mit etwa bis zu insgesamt 24 C-Atomen in den Kohlenstoffresten. Besonders bevorzugt, nicht zuletzt aus wirtschaftlichen Gründen, ist das Triphenylphosphin. Allgemein ist den Phosphinen der Vorzug vor den Phosphiten zu geben, weil letztere mit dem Wasser hydrolysieren und Umlagerungsreaktionen eingehen können.

Die Menge dieser Liganden beträgt im allgemeinen 1 bis 20, vorzugsweise 1 bis 5 mol pro Mol Palladium.

Die Menge des Kohlendioxids, welches die Butadien-Telomerisation auf bisher nicht bekannte Weise begünstigt, ist ebenfalls nicht kritisch und kann etwa $10^{-3}$ bis 1, vorzugsweise $10^{-2}$ bis 0,5 mol pro Mol Butadien betragen.

Für einen vollständigen Umsatz des Butadiens zu den Octadienolen sind mindestens äquimolare Mengen Wasser (d.h. Molverhältnis von Wasser zu Butadien = 0,5:1) erforderlich, jedoch empfiehlt sich ein höheres Molverhältnis von Wasser zu Butadien bis zu etwa 10:1, vorzugsweise 5:1, um die Konkurrenzreaktionen der Bildung von Octadienylethern, Octatrien und höheren Polyenen zu unterdrücken.

Als aprotische polare Lösungsmittel kommen vor allem Ether in Betracht, da diese sich unter den Reaktionsbedingungen inert verhalten und ein hinreichendes bis gutes Lösevermögen sowohl für Butadien als auch für Wasser aufweisen. Genannt seien Diethylether, Tetrahydrofuran und 1,4-Dioxan.

Verfahrenstechnisch besonders empfehlenswert sind solche Lösungsmittel, die höher als die gebildeten Octadienole sieden, weil man in diesem Fall die Octadienole einfach von dem katalysatorhaltigen Lösungsmittel abdestillieren kann. Geeignete hochsiedende Lösungsmittel sind vor allem Polyalkylenglykolether der Formel

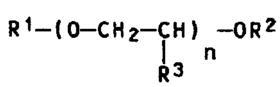

in der $R^1$ und $R^2$ $C_1$-$C_3$ Alkylgruppen und $R^3$ Wasserstoff oder die Methylgruppe bedeuten und n für einen Wert von 2 bis 6, vorzugsweise 4 steht.

Weitere geeignete hochsiedene Lösungsmittel sind Dialkylsulfoxide wie vor allem Dimethylsulfoxid und Sulfone wie Tetrahydrothiophen-1,1-dioxid-(Sulfolan).

Mit Hilfe der hochsiedenen Lösungsmittel läßt sich das erfindungsgemäße Verfahren besonders vorteilhaft kontinuierlich gestalten, indem man die genannten Komponenten in einem Reaktionsgefäß R miteinander umsetzt, das Reaktionsgemisch von R in eine Destillationsapparatur D überführt und dort nicht umgesetztes Butadien sowie die Octadienole fraktioniert als Destillate abtrennt und das Butadien sowie das katalysatorhaltige Sumpfprodukt von D nach R zurückführt.

Man nimmt die Reaktion vorzugsweise bei 50 bis 70 °C vor. Unterhalb von 50 °C, besonders unterhalb von 30 °C, verlangsamt sich die Umsetzung zu sehr, und oberhalb von 90 °C, besonders 100 °C, ist mit unerwünschten Nebenreaktionen zu rechnen, die man möglicherweise dann in Kauf nehmen wird, wenn man auf hohe Raum-Zeit-Ausbeuten großen Wert legt.

Für den Druck empfiehlt sich der Eigendruck bei den gewählten Reaktionstemperaturen; er liegt meistens im Bereich von 5 bis 50, besonders 10 bis 30 bar.

Eine wirtschaftlich sehr vorteilhafte Ausführungsform des erfindungsgemäßen Verfahrens besteht in der Verwendung von sog. $C_4$-Schnitten anstelle von reinem Butadien. Die im $C_4$-Schnitt neben Butadien enthaltenen Olefine But-1-en, But-2-en und Isobuten nehmen weder an der Reaktion teil, noch beeinträchtigen sie diese. Die $C_4$-Schnitte enthalten etwa 45 Gew.% Butadien, 17 Gew.% But-1-en, 10 Gew.% But-1-en, 25 Gew.% Isobuten und den Rest Butan und Isobutan.

Im übrigen weist das erfindungsgemäße Verfahren keine verfahrenstechnischen Besonderheiten auf, so daß nähere Ausführungen hierzu entbehrlich sind.

Das gleiche gilt für die Aufarbeitung des Reaktionsgemisches.

Das erfindungsgemäße Verfahren zeichnet sich dadurch aus, daß man bei hohen Butadienumsätzen von etwa 80 bis 100 % besonders hohe Selektivitäten an Octa-2,7-dien-1-ol erzielt, die im Bereich von etwa 75 bis 90 % liegen, Werte, die nach den bisher bekannten Methoden nicht zu erreichen sind.

Beispiel 1

Auf eine in einem 300 ml Autoklaven befindliche Mischung aus 127 ml eines Lösungsmittels, 30 g (1,67 mol) Wasser, 0,22 g (0,72 mmol) Palladium-(II)-acetylacetonat, 0,56 g (2,16 mmol) Triphenylphosphin und 0,032 g (0,36 mmol) Tetrafluorborsäure wurden unter Argon als Schutzgas 22,5 g (416 mmol) Butadien und 4,4 g (100 mmol) Kohlendioxid aufgepreßt, wonach das Gemisch für 8 Stunden unter Rühren auf 50 °C gehalten wurde.

Danach wurde der flüssige einphasige, leicht gelblich gefärbte, klare Reaktoraustrag mittels Kapillargaschromatographie (interner Standard n-Decanol) analysiert.

Die gleichen Versuche wurden ohne Mitverwendung der Säure $HBF_4$ wiederholt (Vergleichsversuche 1a und 2a). Weitere Einzelheiten zu diesen Versuchen sowie deren Ergebnisse sind Tabelle 1 zu entnehmen.

Tabelle 1

| Versuch Nr. | Lösungsmittel | Umsatz [%] | Selektivität [mol%] | | |
|---|---|---|---|---|---|
| | | | $C_8$-2,7-dien-1-ol | $C_8$-1,7-dien-3-ol | $C_8$ und $C_{12}$-Polyene |
| 1 | Dimethylsulfoxid | 88 | 86,5 | 3,9 | 4,4 |
| 1a)* | " | 83 | 74,7 | 4,0 | 15,9 |
| 2 | Tetraethylenglykoldimethylether | 95 | 79,5 | 7,9 | 8,1 |
| 2a)* | " | 62 | 81,4 | 7,9 | 7,6 |

*) ohne $HBF_4$

Beispiel 2

Auf die in Versuch 2, Beispiel 1, angegebenen Weise, jedoch mit 0,7 g (16 mmol) Kohlendioxid und bei 60 °C wurde ein Reaktionsgemisch hergestellt, welches anschließend zur Abtrennung der Verfahrensprodukte der Dünnschichtdestillation bei 100 °C und 4 mbar unterworfen wurde.

Das katalysatorhaltige Sumpfprodukt wurde sodann mit denjenigen Mengen frischer Einsatzstoffe versetzt, die bei der Destillation mitentfernt wurden und für einen weiteren gleichartigen Versuch wiederverwendet. Insgesamt wurde dieser Zyklus fünfmal wiederholt. Aus den in Tabelle 2 zusammengestellten Ergebnissen ist zu ersehen, daß die Katalysatoraktivität bei wiederholtem Einsatz der Sumpfphase praktisch konstant blieb.

Tabelle 2

| Versuch Nr. | Umsatz [%] | Selektivität [mol%] $C_8$-2,7-dien-1-ol | $C_8$-1,7-dien-3-ol | $C_8$ + $C_{12}$-Polyene |
|---|---|---|---|---|
| 1 | 88 | 77,2 | 8,7 | 10,4 |
| 2 | 90 | 81,7 | 5,9 | 7,1 |
| 3 | 82 | 77,8 | 6,0 | 8,9 |
| 4 | 92 | 81,6 | 4,9 | 7,6 |
| 5 | 85 | 85,4 | 5,5 | 8,1 |
| 6 | 88 | 86,1 | 6,6 | 4,8 |

Beispiel 3

Auf die im Versuch 2 von Beispiel 1 angegebene Weise, jedoch mit 15 g (0,83 Mol) Wasser, wurden verschiedene nicht-koordinierende Säuren im Äquivalenzverhältnis von Säure zu Palladium von 0,5:1 auf ihren katalytischen Effekt untersucht.

Die Einzelheiten dieser Versuche sowie deren Ergebnisse sind Tabelle 3 zu entnehmen.

Tabelle 3

| Versuch Nr. | Säure | Umsatz [mol%] | Selektivität [mol%] $C_8$-2,7-dien-1-ol | $C_8$-1,7-dien-3-ol | $C_8$ + $C_{12}$-Polyene |
|---|---|---|---|---|---|
| 1 | $HBF_4$ | 100 | 79,5 | 10,3 | 6,1 |
| 2 | $HPF_6$ | 81 | 80,5 | 6,5 | 6,9 |
| 3 | p-$CH_3$-Ph-$SO_3H$ b) | 72 | 75,7 | 12,0 | 7,8 |
| 4 | $H_2SO_4$ | 91 | 69,4 | 15,4 | 9,4 |
| 5 | $CF_3CO_2H$ | 77 | 59,9 | 23,1 | 7,9 |
| 6a *) | - | 73 | 63,4 | 22,5 | 11,9 |

*) zum Vergleich ohne Säure
b) p-Toluolsulfonsäure

Beispiel 4

Der Einfluß des Äquivalenzverhältnisses von Säure zu Palladium geht aus den Versuchen von Tabelle 4 hervor, die analog Versuch 2 von Beispiel 1, jedoch mit 15 g (0,83 mol) Wasser vorgenommen wurden.

Tabelle 4

| Versuch Nr. | $HBF_4$/Pd | Umsatz [%] | Selektivität [mol%] $C_8$-2,7-dien-1-ol | $C_8$-2,7-dien-3-ol | $C_8$ + $C_{12}$ Polyene |
|---|---|---|---|---|---|
| 1*) | 0 | 73 | 63,4 | 22,5 | 11,9 |
| 2 | 25 | 74 | 72,4 | 17,3 | 8,7 |
| 3 | 50 | 100 | 79,5 | 10,3 | 6,1 |
| 4 | 75 | 84 | 82,3 | 6,8 | 4,4 |
| 5 | 100 | 70 | 80,2 | 5,7 | 8,0 |

*) ohne Säurezusatz

Beispiel 5

Die eingesetzten Mengen und der Ablauf entsprechen den Versuchsbedingungen in Beispiel 1, Versuch 2, abweichend davon wurden 15 g (0,83 Mol) Wasser eingesetzt.

Das Butadien wurde jedoch in Form eines $C_4$-Schnittes eingesetzt, welcher durch Zumischen von Raffinat I, bestehend aus 29 % But-1-en, 16 % But-2-en, 45 % Isobuten und 10 % Butane hergestellt wurde. Zum Vergleich wurde eine Umsetzung mit reinem Butadien (Versuch 2) durchgeführt, die verdeutlicht, daß die übrigen Alkene im $C_4$-Schnitt die Butadientelomerisation nicht beeinträchtigen.

Tabelle 5

| Versuch Nr. | Temp. [°C] | Butadien in $C_4$-Schnitt[%] Einsatz | Austrag | Umsatz [%] | Selektivität [mol %] $C_8$-2,7-dien-1-ol | $C_8$-1,7-dien-3-ol | $C_8$ + $C_{12}$-Polyene |
|---|---|---|---|---|---|---|---|
| 1 | 50 | 38 | 2,5 | 94 | 78,2 | 4,3 | 7,0 |
| 2*) | 50 | 100 | - | 99 | 78,0 | 7,9 | 8,3 |
| 3 | 60 | 45 | 7,6 | 86 | 81,1 | 6,5 | 6,5 |
| 4 | 70 | 45 | 2,3 | 90 | 77,0 | 4,9 | 10,1 |

*) Einsatz von reinem Butadien (Vergleich)

## Patentansprüche

1. Verfahren zur Herstellung von Octadienolen durch Telomerisation von Buta-1,3-dien mit Wasser in Gegenwart von Kohlendioxid und eines Katalysatorsystems aus einer Palladiumverbindung und einer tertiären Phosphorverbindung, dadurch gekennzeichnet, daß man es in Gegenwart einer Säure ausgewählt aus der Gruppe enthaltend Tetrafluorborsäure, Hexafluorphosphorsäure, Methansulfonsäure, Trifluormethansulfonsäure, p-Toluolsulfonsäure, Schwefelsäure, Trifluoressigsäure, Trichloressigsäure und Essigsäure durchführt, wobei die Konzentration der Säure 0,1 bis 150 Äquivalent-% pro Mol Palladium beträgt.

2. Verfahren nach Anspruch 1, dadurch gekennzeichnet, daß man es in Gegenwart eines aprotischen polaren Lösungsmittels vornimmt, in welchem Wasser und Buta-1,3-dien mindestens teilweise löslich sind.

3. Verfahren nach Anspruch 2, dadurch gekennzeichnet, daß das Lösungsmittel einen höheren Siedepunkt als die hergestellten Octadienole hat.

4. Verfahren nach Anspruch 3, dadurch gekennzeichnet, daß man als Lösungsmittel Polyalkylenglykolether einsetzt.

5. Verfahren nach Anspruch 3, dadurch gekennzeichnet, daß man als Lösungsmittel Sulfoxide oder Sulfone einsetzt.

6. Verfahren nach den Ansprüchen 1 bis 5, dadurch gekennzeichnet, daß man das Buta-1,3-dien in Form eines Gemisches mit anderen gesättigten oder olefinischen $C_4$-Kohlenwasserstoffen einsetzt.

7. Verfahren zur Herstellung von Octadienolen durch Telomerisation von Buta-1,3-dien mit Wasser in Gegenwart von Kohlendioxid und eines Katalysatorsystems aus einer Palladiumverbindung und einer tertiären Phosphorverbindung, dadurch gekennzeichnet, daß man es in Gegenwart Säure ansgewählt aus der Gruppe enthaltend Tetrafluorborsäure, Hexafluorphosphonsäure, Methansulfonsäure, Trifluormethansulfonsäure, p-Toluolsulfonsäure, Schwefelsäure, Trifluoressigsäure, Trichloressigsäure und Essigsäure sowie eines aprotischen, polaren Lösungsmittels, in welchem Wasser und Buta-1,3-dien zumindest teilweise löslich sind und welches höher als die entstehenden Octadienole siedet, in der Weise kontinuierlich vornimmt, daß man
   a) die genannten Komponenten in einem Reaktionsgefäß R miteinander umsetzt,
   b) das Reaktionsgemisch von R in eine Destillationsapparatur D überführt und dort nicht umgesetztes Buta-1,3-dien sowie die Octadienole fraktioniert als Destillate abtrennt und
   c) das Buta-1,3-dien sowie das katalysatorhaltige Sumpfprodukt von D nach R zurückführt.

## Claims

1. A process for preparing an octadienol by telomerizing 1,3-butadiene with water in the presence of carbon dioxide and a catalyst system comprising a palladium compound and a tertiary phosphorus compound, which is carried out in the presence of an acid selected from the group consisting of tetrafluoroboric acid, hexafluorophosphoric acid, methanesulfonic acid, trifluoromethanesulfonic acid, p-toluenesulfonic acid, sulfuric acid, trifluoroacetic acid, trichloroacetic acid and acetic acid, being the concentration of the acid from 0.1 to 150 equivalent-% per mole of palladium.

2. A process as claimed in claim 1, which is carried out in the presence of an aprotic polar solvent in which water and 1,3-butadiene are at least partially soluble.

3. A process as claimed in claim 2, wherein the solvent has a higher boiling point than the octadienol prepared.

4. A process as claimed in claim 3, wherein the solvent used is polyalkylene glycol ether.

5. A process as claimed in claim 3, wherein the solvent used is a sulfoxide or sulfone.

6. A process as claimed in any of claims 1 to 5, wherein the 1,3-butadiene is used in the form of a mixture with another saturated or olefinic $C_4$ hydrocarbon.

7. A process for preparing an octadienol by telomerizing 1,3-butadiene with water in the presence of carbon dioxide and a catalyst system comprising a palladium compound and a tertiary phosphorus compound, which is carried out in the presence of an acid selected from the group consisting of tetrafluoroboric acid, hexafluorophosphoric acid, methanesulfonic acid, trifluoromethanesulfonic acid, p-toluenesulfonic acid, sulfuric acid, trifluoroacetic acid, trichloroacetic acid and acetic acid and of an aprotic polar solvent in which water and 1,3-butadiene are at least partially soluble and which has a higher boiling point than the octadienol formed in a continuous form by
   a) making the components react with one another in a reaction vessel R,
   b) transferring the reaction mixture from R to a distillation apparatus D where unconverted 1,3-butadiene and the octadienols are separated off as distillates by fractional distillation, and
   c) recycling 1,3-butadiene and a catalyst-containing bottom product from D into R.

## Revendications

1. Procédé de fabrication d'octadiénols par la télomérisation du buta-1,3-diène avec de l'eau, en présence de dioxyde de carbone et d'un système catalytique constitué d'un composé du palladium et d'un composé du phosphore tertiaire, caractérisé en ce qu'on l'entreprend en présence d'un acide choisi dans le groupe formé par l'acide tétrafluoroborique, l'acide hexafluorophosphorique, l'acide méthanesulfonique, l'acide trifluorométhanesulfonique, l'acide p-toluènesulfonique, l'acide sulfurique, l'acide trifluoracétique, l'acide trichloracétique et l'acide acétique et la concentration de l'acide varie de 0,1 à 150%-équivalents par mole de palladium.

2. Procédé suivant la revendication 1, caractérisé en ce qu'on l'entreprend en présence d'un solvant polaire et aprotique, dans lequel l'eau et le buta-1,3-diène sont au moins partiellement solubles.

3. Procédé suivant la revendication 2, caractérisé en ce que le solvant possède un point d'ébullition supérieur à celui des octadiénols fabriqués.

4. Procédé suivant la revendication 3, caractérisé en ce que l'on utilise des polyalkylèneglycoléthers à titre de solvants.

5. Procédé suivant la revendication 3, caractérisé en ce que l'on utilise des sulfoxydes ou des sulfones à titre de solvant.

6. Procédé suivant l'une quelconque des revendications 1 à 5, caractérisé en ce que l'on utilise le buta-1,3-diène sous la forme d'un mélange avec d'autres hydrocarbures en $C_4$, saturés ou oléfiniques.

7. Procédé de fabrication d'octadiénols par la télomérisation du buta-1,3-diène avec de l'eau, en présence de dioxyde de carbone et d'un système catalytique constitué d'un composé du palladium et d'un composé du phosphore tertiaire, caractérisé en ce qu'on l'entreprend en présence d'un acide choisi dans le groupe formé par l'acide tétrafluoroborique, l'acide hexafluorophosphorique, l'acide méthanesulfonique, l'acide trifluorométhanesulfonique, l'acide p-toluènesulfonique, l'acide sulfurique, l'acide trifluoracétique, l'acide trichloracétique et l'acide acétique, comme aussi d'un solvant polaire et aprotique dans lequel l'eau et le buta-1,3-diène sont au moins partiellement solubles et qui bout à une température supérieure au point d'ébullition des octadiénols qui se forment et il se déroule en continu et on opère de façon à ce que
   a) on fasse réagir des composants précités les uns avec les autres dans un récipient de réaction R,
   b) on transfère le mélange réactionnel de R dans un appareil de distillation D où l'on sépare le buta-1,3-diène non converti comme aussi les octadiénols fractionnés sous forme de distillat et
   c) on renvoie le buta-1,3-diène, comme aussi le produit de fond contenant du catalyseur de D vers R.